(19) Europäisches Patentamt / European Patent Office / Office européen des brevets

(11) **EP 4 454 606 A1**

(12) **EUROPEAN PATENT APPLICATION**
published in accordance with Art. 153(4) EPC

(43) Date of publication:
**30.10.2024 Bulletin 2024/44**

(21) Application number: **22909703.5**

(22) Date of filing: **02.12.2022**

(51) International Patent Classification (IPC):
***A61F 2/07*** *(2013.01)*

(52) Cooperative Patent Classification (CPC):
**A61F 2/07; A61F 2/962; A61F 2/966; A61M 25/00**

(86) International application number:
**PCT/CN2022/136076**

(87) International publication number:
**WO 2023/116388 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.12.2021 CN 202111573206**

(71) Applicant: **Lifetech Scientific (Shenzhen) Co., Ltd.
Shenzhen, Guangdong 518000 (CN)**

(72) Inventors:
• **TANG, Jiangfeng
Shenzhen, Guangdong 518000 (CN)**
• **XIAO, Benhao
Shenzhen, Guangdong 518000 (CN)**

(74) Representative: **Michalski Hüttermann & Partner
Patentanwälte mbB
Kaistraße 16A
40221 Düsseldorf (DE)**

(54) **DELIVERY SYSTEM**

(57) The present invention relates to a delivery system (100), including an outer sheath assembly (1), an inner sheath assembly (3), and a sealing assembly (5), where the outer sheath assembly (1) includes an outer sheath core tube (11); the inner sheath assembly (3) includes an inner sheath core (31) arranged in the outer sheath core tube (11) in a penetrating manner; the sealing assembly (5) includes a first sealing piece (51); and the first sealing piece (51) is arranged between the inner sheath core (31) and the outer sheath core tube (11) to seal a gap between a proximal end of the outer sheath core tube (11) and the inner sheath core (31). The delivery system can effectively block blood flowing into a gap between the outer sheath core tube (11) and the inner sheath core (31), so that the blood is avoided from flowing out of a product, thereby solving the technical problem of blood leakage of the product.

FIG. 1

EP 4 454 606 A1

# Description

## Technical Field

The present embodiments belongs to the technical field of medical instruments, and particularly relates to a delivery system.

## Background Art

A minimally invasive surgery for vascular repair with a covered stent is widely used for treating aortic vascular diseases due to its minimal injury, rapid recovery and good immediate effects. During clinical implantation, the covered stent is pre-mounted into a sheath tube of a delivery system firstly. Then, the covered stent passes through a human vascular lumen, and is delivered to a lesion location by the sheath tube of the delivery system. Finally, the covered stent is released from the sheath tube of the delivery system. The covered stent isolates a blood flow from the lesion location of a blood vessel, so as to achieve a purpose of treatment. Due to a gap between an outer wall of an inner sheath core and an inner cavity of an outer sheath core tube of the delivery system, blood is liable to leak from the gap, to cause a blood leakage phenomenon during a surgery, which causes a certain damage on a patient.

## Summary of the Invention

An objective of the present disclosure is to provide a delivery system, aiming to solve the technical problem that a blood leakage phenomenon occurs due to poor sealing effects of an existing delivery system, to cause damage on a patient.

The present disclosure is realized as follows: the delivery system includes an outer sheath assembly, an inner sheath assembly, and a sealing assembly, where the outer sheath assembly includes an outer sheath core tube; the inner sheath assembly includes an inner sheath core arranged in the outer sheath core tube in a penetrating manner; and the sealing assembly includes a first sealing piece, and the first sealing piece is arranged between the inner sheath core and the outer sheath core tube to seal a gap between a proximal end of the outer sheath core tube and the inner sheath core.

The present disclosure has the beneficial effects that: for the delivery system of the present disclosure, a first sealing piece is arranged between an inner sheath core and an outer sheath core tube. The gap between the proximal end of the outer sheath core tube and the inner sheath core is sealed by the first sealing piece, which can effectively block blood flowing into the gap. Therefore, the blood is avoided from flowing out of a product during a surgery, and the technical problem of blood leakage of the product is solved.

## Brief Description of the Drawings

FIG. 1 is a longitudinal cross-sectional view of a delivery system of an embodiment I;

FIG. 2 is a partial enlarged view A in FIG. 1;

FIG. 3 is a partial enlarged view B in FIG. 1;

FIG. 4 is a longitudinal cross-sectional view of a delivery system of an embodiment II ;

FIG. 5 is a partial enlarged view C in FIG. 4;

FIG. 6 is a longitudinal cross-sectional view of a sealing tube of an embodiment II;

FIG. 7 is a longitudinal cross-sectional view of a delivery system of an embodiment III;

FIG. 8 is a partial enlarged view D in FIG. 7;

FIG. 9 is a longitudinal cross-sectional view I of an outer core lock sealing gasket of the delivery system of the embodiment III;

FIG. 10 is a longitudinal cross-sectional view II of the outer core lock sealing gasket of the delivery system of the embodiment III;

FIG. 11 is a partial longitudinal cross-sectional view of a delivery system of an embodiment IV;

FIG. 12 is a partial enlarged view E in FIG. 11;

FIG. 13 is a partial longitudinal cross-sectional view of a delivery system of an embodiment V; and

FIG. 14 is a longitudinal cross-sectional view of a push rod sealing gasket of a delivery system of an embodiment V.

100-delivery system,

1-outer sheath assembly,

11-outer sheath core tube, 111-inner tube, 113-outer tube,

13-proximal releaser, 131-mounting hole,

15-second connecting component, 151-outer core grasper, 1511-jaw, 153-outer core lock, 1531-through hole,

17-cavity,

3-inner sheath assembly,

31-inner sheath core,

33-rear connecting piece, 331-through hole,

35-first connecting component, 351, Luer lock, 3511-proximal sealing piece, 353-inner core grasper,

5-sealing assembly,

51-first sealing piece, 51a, sealing ring, 51b-sealing tube, 51 1b-guide piece, 51c-proximal releaser sealing gasket, 51d-outer core lock sealing gasket,

53-second sealing piece, 53a-bulge, 53b-push rod sealing gasket, 531b- void avoidance groove,

7-push rod assembly,

71-push rod,

73-push rod joint,

75-third connecting component,

77-rear handle, 771-clamping groove,

9-guide rod.

## Detailed Description of the Invention

In order to make the objectives, technical solutions and advantages of the present disclosure clearer, the present disclosure will be further described in detail below in conjunction with the drawings and embodiments. It should be understood that the specific embodiments described herein are merely illustrative of the present disclosure and are not intended to limit the present disclosure.

It should be noted that when an element is considered to be "fixed" or "arranged" to another element, the element may be directly arranged on another element or there may be intermediate element simultaneously. When an element is considered to be "connected" to another element, the element may be directly connected to another element or there may be an intermediate element simultaneously.

It should also be noted that the left, right, upper, lower and other orientation terms in the embodiments are only relative concepts or refer to the normal use status of the product, and should not be considered as limiting.

It should be noted that in the field of invasive medical instruments, it is general to refer one end, closer to an operator, of a medical instrument implanted into a human body or an animal body to as the "proximal end", and one end farther from the operator to as the "distal end". Based on this principle, the "proximal end" and the "distal end" of any component of the medical instrument are defined. An "axial direction" generally refers to a length direction of the medical instrument when it is delivered; and a "radial direction" generally refers to a direction perpendicular to the "axial direction" of the medical instrument. Based on this principle, the "axial direction" and the "radial direction" of any component of the medical instrument are defined.

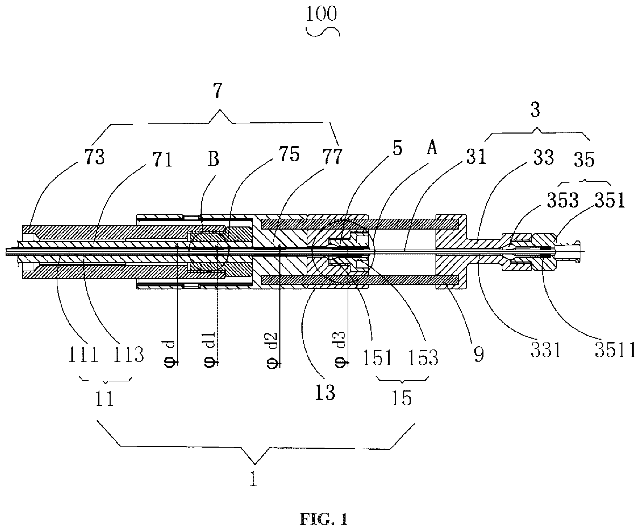

As shown in FIG. 1, a delivery system 100 provided by an embodiment I of the present embodiment includes: an outer sheath assembly 1, an inner sheath assembly 3, a sealing assembly 5, a push rod assembly 7, a guide rod 9 and a sheath tube (not shown in the FIG. 1). The sheath tube is used for at least receiving distal ends of the outer sheath assembly 1, the inner sheath assembly 3, and the push rod assembly 7.

The outer sheath assembly 1 includes an outer sheath core tube 11, a proximal releaser 13 and a second connecting component 15. The outer sheath core tube 11 is fixed to the proximal releaser 13 by the second connecting component 15. The outer sheath core tube 11, the proximal releaser 13 and the second connecting component 15 are assembled to form an integral structure.

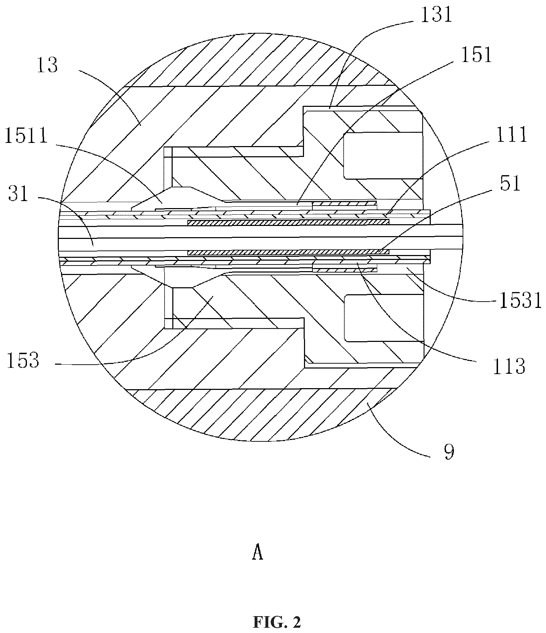

As shown in FIGS. 1 and 2, in the embodiment, the outer sheath core tube 11 includes an inner tube 111 and an outer tube 113 sleeving outside the inner tube 111. Hardness of the outer tube 113 is larger than that of the inner tube 111. An axial length of the inner tube 111 is roughly the same as that of the outer tube 113. In the embodiment, the inner tube 111 may be a PI tube; the outer tube 113 is a stainless steel tube; and the inner tube 111 is fixedly connected to the outer tube 113 in an adhesion way. Specifically, the inner tube 111 is adhered into the outer tube 113 by glue. The outer tube 113 sleeving outside the inner tube 111 may effectively protect the inner tube 111 due to its larger hardness than the inner tube 111, so as to avoid a deformation of the inner tube 111 due to excessive force from the second connecting component 15 in grasping the outer sheath core tube 11.

A mounting hole 131 used for storing the second connecting component 15 is formed in a proximal end of the proximal releaser 13. A shape of the mounting hole 131 corresponds to that of the second connecting component 15. Threads used for connection are formed in an inner wall of the mounting hole 131.

The second connecting component 15 includes an outer core lock 153 and an outer core grasper 151. External threads connected to the inner wall of the mounting hole 131 are formed in a distal end of the outer core lock 153. The distal end of the outer core lock 153 is mounted in the mounting hole 131 of the proximal releaser 13. The outer core lock 153 is in threaded connection with the proximal releaser 13. A through hole 1531 is formed in the outer core lock 153 in an axial direction. A proximal end of the outer core grasper 151 is inserted into the through hole 1531 of the outer core lock 153. The outer core grasper 151 is capable of axially moving relative to the outer core lock 153. A jaw 1511 is arranged at a distal end of the outer core grasper 151, and may deform under external force. The outer core grasper 151 sleeves outside the outer sheath core tube 11. The jaw 1511 grasps the outer tube 113. Inner walls of the outer core lock 153 and the proximal releaser 13 are both provided with an inclined surface making a contact with the jaw 1511. The outer core lock 153 drives the outer core grasper 151 to fix the outer sheath core tube 11 to the proximal releaser 13. Specifically, when the outer core lock 153 is axially screwed into the proximal releaser 13, the outer core lock 153 and the proximal releaser 13 make a contact with the jaw 1511, respectively. The outer core lock 153 and the proximal releaser 13 apply radial force to the jaw 1511. The jaw 1511 firmly grasps the outer tube 113.

As shown in FIG. 1, the inner sheath assembly 3 includes an inner sheath core 31, a rear connecting piece 33 and a first connecting component 35. The inner sheath core 31 is fixed to the rear connecting piece 33 by the first connecting component 35. The inner sheath core 31, the rear connecting piece 33 and the first connecting component 35 are assembled to form an integral structure.

The inner sheath core 31 is arranged in the outer sheath core tube 11 in a penetrating manner. An axial length of the inner sheath core 31 is larger than that of the outer sheath core tube 11. The outer sheath core tube 11 sleeves outside a part of the inner sheath core 31.

A through hole 331 penetrating in the axial direction is formed in the rear connecting piece 33. The inner sheath core 31 is arranged in the through hole 331 in a penetrating manner. The rear connecting piece 33 is fixedly connected to the first connecting component 35. In the embodiment, a connection way between the rear connecting piece 33 and the first connecting component 35 is the same as that between the proximal releaser 13 and the second connecting component 15 in the outer sheath assembly 1. A proximal end of the rear connecting piece 33 is in threaded connection with the first connecting component 35.

The first connecting component 35 includes a Luer lock 351 and an inner core grasper 353. A proximal sealing piece 3511 is mounted in the Luer lock 351, and is arranged at a proximal end of the first connecting component 35. The proximal sealing piece 3511 may provide a sealing effect for a pre-surgery exhaust operation. External threads connected to the rear connecting piece 33 are formed in a distal end of the Luer lock 351. The distal end of the Luer lock 351 is inserted into the proximal end of the rear connecting piece 33. The Luer lock 351 is in threaded connection with the rear connecting piece 33. A shape, a fixing way and a working principle of the inner core grasper 353 are the same as those of the outer core grasper 151. A proximal end of the inner core grasper 353 is inserted into the Luer lock 351. The inner core grasper 353 is capable of axially moving relative to the Luer lock 351. A jaw that may deform under the external force is arranged at a distal end of the inner core grasper 353. The inner core grasper 353 sleeves outside the inner sheath core 31. The jaw grasps the inner sheath core 31. Inner walls of the Luer lock 351 and the rear connecting piece 33 are both provided with an inclined surface making a contact with the jaw. When the Luer lock 351 is screwed into the rear connecting piece 33 in an axial direction, the Luer lock 351 and the rear connecting piece 33 apply radial force to the jaw, and the jaw firmly grasps the inner sheath core 31. The inner sheath core 31 is fixed to the rear connecting piece 33 by the first connecting component 35.

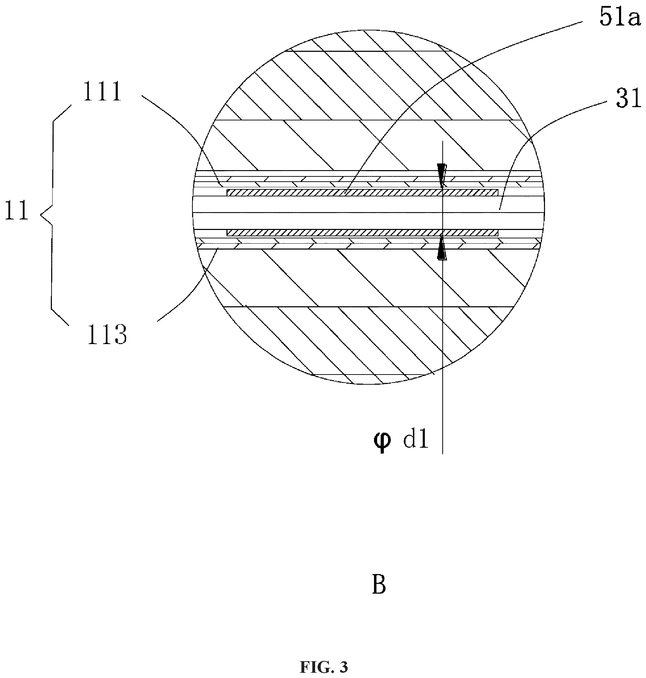

As shown in FIGS. 1-3, in the embodiment, the sealing assembly 5 includes a first sealing piece 51. The first sealing piece 51 is arranged between the inner sheath core 31 and the outer sheath core tube 11. The first sealing piece 51 is used for sealing a gap between a proximal end of the outer sheath core tube 11 and the inner sheath core 31, so as to avoid blood from flowing out along the gap between the outer sheath core tube 11 and the inner sheath core 31 during a surgery. The first sealing piece 51 extends in a length direction of the outer sheath core tube 11. At least part of the first sealing piece 51 is arranged between the outer sheath core tube 11 and the inner sheath core 31.

The first sealing piece 51 may be of a plurality of structures. In the embodiment, the first sealing piece 51 includes at least one sealing ring 51a. The sealing ring 51a is arranged between the outer sheath core tube 11 and the inner sheath core 31. Specifically, the sealing ring 51a is arranged between an outer wall of the inner sheath core 31 and an inner wall of the inner tube 111. The sealing ring 51a is connected to the inner sheath core 31 or the outer sheath core tube 11. In the embodiment, the sealing ring 51a is fixedly connected to the inner sheath core 31 by gluing or welding or in other ways. In order to improve the sealing effect, a plurality of sealing rings 51a are arranged in the embodiment. Axial lengths of the plurality of sealing rings 51a are smaller than the axial length of the inner sheath core 31. The plurality of sealing rings 51a discontinuously extend in the length direction of the outer sheath core tube 11.

The sealing rings 51a sleeve outside the inner sheath core 31. The plurality of sealing rings 51a are arranged in an axial direction of the inner sheath core 31. Outside diameter dimensions of the plurality of sealing rings 51a change regularly. Specifically, from a distal end of the inner sheath core 31 to a proximal end of the inner sheath core 31, radial distances between the sealing rings 51a and the outer sheath core tube 11 are gradually decreased. That is, from the distal end of the inner sheath core 31 to the proximal end of the inner sheath core 31, outer walls of the sealing rings 51a gradually approach an inner wall of the outer sheath core tube 11. As shown in FIG. 1, an inside diameter of the outer sheath core tube 11 is d. Three sealing rings 51a are sequentially arranged from a distal end of the outer sheath core tube 11 to the proximal end of the outer sheath core tube 11.

Outside diameters of the three sealing rings 51a are an outside diameter d1, an outside diameter d2, and an outside diameter d3. An inside diameter d of the outer sheath core tube 11, and the outside diameter d1, the outside diameter d2, and the outside diameter d3 of the three sealing rings 51a satisfy the following relationship:

$$\varnothing d > \varnothing d3 > \varnothing d2 > \varnothing d1$$

The outside diameter d1, the outside diameter d2, and the outside diameter d3 of the three sealing rings 51a are gradually increased, but always smaller than the inside diameter d of the outer sheath core tube 11.

Gaps between the outer walls of the sealing rings 51a and the inner wall of the outer sheath core tube 11 are gradually decreased. There is a certain interval between every two sealing rings 51a. The purpose of this design is that if the blood flows through the first sealing ring 51a with the outside diameter d1, a blood flow volume is decreased by resistance due to decrease in the gap. After the blood flow passes through a normal gap section, pressure of the blood flow is decreased at this time; and then after the blood passes through a next sealing ring 51a with a smaller gap, the blood flow volume and the pressure of the blood flow are further decreased. Through obstruction of the plurality of different sealing rings 51a, the blood flow volume and the pressure of the blood flow are gradually decreased, so as to achieve the sealing effect. By decreasing the gaps between local positions of the outer sheath core tube 11 and the inner sheath core 31, such sealing structure can not only achieve the good sealing effect, but also not significantly strengthen resistance on axial movement of the outer sheath core tube 11 relative to the inner sheath core 31.

As shown in FIG. 1, the push rod assembly 7 includes a push rod 71, a push rod joint 73, a third connecting component 75 and a rear handle 77.

The outer sheath core tube 11 is arranged in the push rod 71 in a penetrating manner. An axial length of the push rod 71 is larger than that of the outer sheath core tube 11.

The push rod joint 73 sleeves outside the push rod 71. An axial length of the push rod joint 73 is smaller than that of the push rod 71. A connecting structure fixed with the third connecting component 75 is arranged at a proximal end of the push rod joint 73. Usually, a threaded structure for connection is arranged at the proximal end of the push rod joint 73.

The third connecting component 75 sleeves outside the push rod 71. An axial length of the third connecting component 75 is smaller than that of the push rod 71. External threads fitted to the push rod joint 73 are formed in the third connecting component 75. The third connecting component 75 is screwed into the push rod joint 73, and is in threaded connection with the push rod joint 73 to form an outer sleeve. An axial length of the outer sleeve is roughly the same as that of the push rod 71.

A proximal end of the push rod 71 is inserted into the third connecting component 75. The proximal end of the push rod 71 is fixedly connected to an inner wall of the third connecting component 75. The push rod 71 is fixed to the push rod joint 73 by the third connecting component 75. The push rod 71, the push rod joint 73 and the third connecting component 75 are assembled to form an integral structure.

The rear handle 77 includes an accommodating cavity. The push rod 71, the push rod joint 73 and the third connecting component 75 are arranged in the accommodating cavity of the rear handle 77. The rear handle 77 is connected to the third connecting component 75. A proximal end of the third connecting component 75 and the proximal end of the push rod 71 are both abutted to the rear handle 77.

As shown in FIG. 1, the guide rod 9 is used for connecting the outer sheath assembly 1, the inner sheath assembly 3 and the push rod assembly 7. A proximal end of the guide rod 9 is inserted into the rear connecting piece 33 of the inner sheath assembly 3, and is fixedly mounted in the rear connecting piece 33. A middle part of the guide rod 9 is arranged in the proximal releaser 13 of the outer sheath assembly 1 in a penetrating manner. The proximal releaser 13 is assembled on the guide rod 9. Under the external force, the proximal releaser 13 may slide in an axial direction of the guide rod 9. A distal end of the guide rod 9 is inserted into the rear handle 77 of the push rod assembly 7, and is fixedly mounted in the rear handle 77. The rear handle 77 is fixedly connected to the rear connecting piece 33 by the guide rod 9.

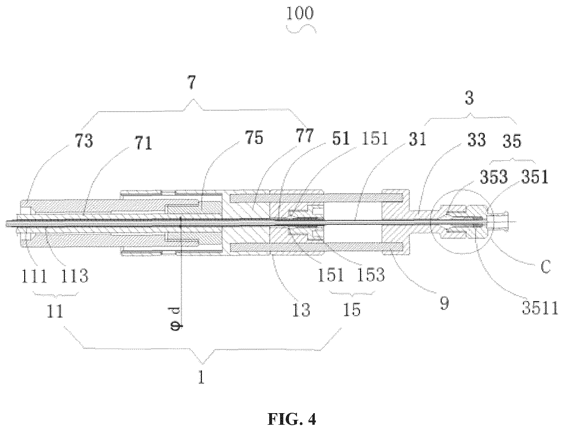

As shown in FIG. 4, a delivery system 100 provided by an embodiment II of the present embodiment includes: an outer sheath assembly 1, an inner sheath assembly 3, a first sealing piece 51, a push rod assembly 7, and a guide rod 9. The embodiment II is different from the embodiment I mainly in a specific structure of the first sealing piece 51.

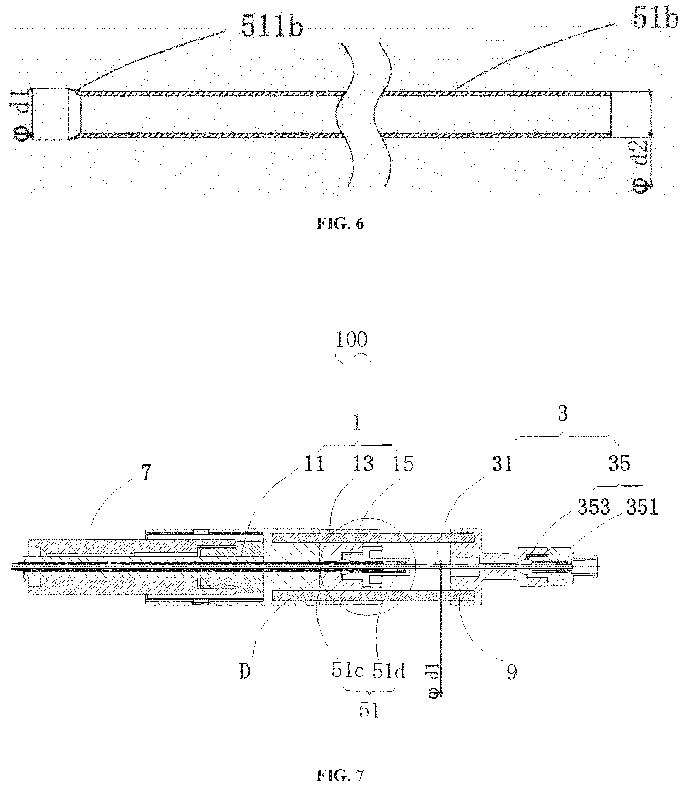

As shown in FIGS. 4 and 6, in the embodiment, the first sealing piece 51 includes a sealing tube 51b. The sealing tube 51b is arranged in an outer sheath core tube 11 in a penetrating manner, and extends continuously in a length direction of the outer sheath core tube 11. The sealing tube 5 1b is arranged between an outer wall of an inner sheath core 31 and an inner wall of an inner tube 111.

A guide piece 511b is arranged at a distal end of the sealing tube 51b, and is used for introducing blood flow into a space between the inner sheath core 31 and the sealing tube 51b. The guide piece 511b is in a shape of a horn mouth. From the distal end of the sealing tube 51b to a proximal end of the sealing tube 51b, an inside diameter of the guide piece 511b is gradually decreased. Specifically, a diameter of a distal end of the guide piece 511b is d1, an outside diameter of the sealing tube 51b is d2, and an inside diameter of the outer sheath core tube 11 is d, where the inside diameter d of the outer sheath core tube 11, the diameter d1 of the distal end of the guide piece 511b, and the outside diameter d2 of the sealing tube 51b satisfy the following relationship:

$$\emptyset d1 > \emptyset d > \emptyset d2, \text{ and } \emptyset d1 < 1.5\emptyset d2$$

The distal end of the guide piece 511b is in interference fitting to the outer sheath core tube 11. The diameter d1 of the distal end of the guide piece 511b should be slightly larger than the inside diameter d of the outer sheath core tube 11, but it should not be too large, which may lead to difficulty of assembly and strengthen resistance on axial movement of the outer sheath core tube 11. The outside diameter d2 of the sealing tube 51b should be smaller than the inside diameter d of the outer sheath core tube 11, so that the outer sheath core tube 11 can move smoothly in an axial direction.

The proximal end of the sealing tube 5 1b is connected to a proximal end of the inner sheath core 31. Hardness of the sealing tube 51b is larger than that of silica gel; and the distal end of the guide piece 511b makes a line contact with an inner cavity of the outer sheath core tube 11 to form interference fitting, thereby improving support force and sealing performance of the guide piece 511b to the outer sheath core tube 11, and avoiding the distal end of the guide piece 511b from being squeezed to deform to weaken flow guide efficiency.

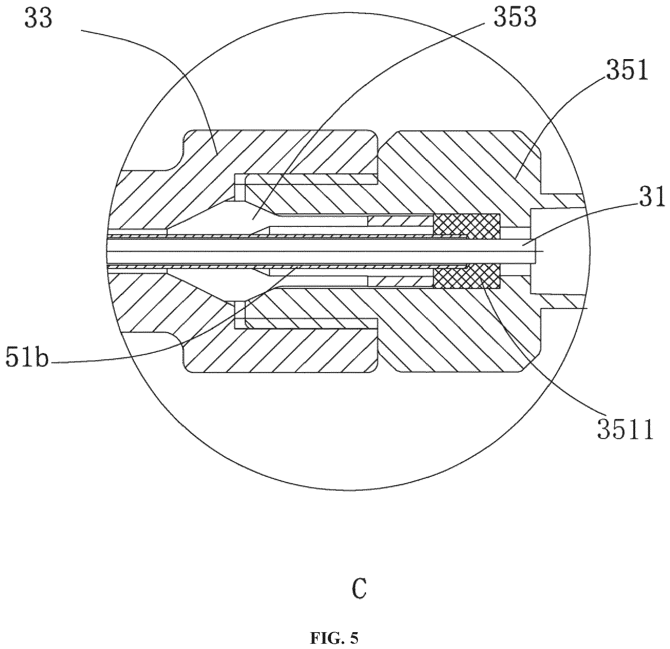

As shown in FIGS. 4 and 5, the distal end of the sealing tube 51b is in interference fitting to the outer sheath core tube 11. The proximal end of the sealing tube 51b penetrates through a rear connecting piece 33 to be in sealing connection with a proximal sealing piece 3511 of a first connecting component 35. Specifically, the proximal end of the sealing tube 51b penetrates through the rear connecting piece 33 to be inserted into the proximal sealing piece 3511.

In the embodiment, when the blood flows to the guide piece 511b, a pathway is not blocked. Instead, the blood flow is introduced into a gap between the inner sheath core 31 and the inner cavity of the sealing tube 51b by the guide piece 511b of the sealing tube 51b. The sealing tube 51b and the inner sheath core 31 are relatively static components in the delivery system 100. A tail end of the sealing tube 51b is inserted into the proximal sealing piece 3511, so as to achieve a sealing effect. In the embodiment, the proximal sealing piece 3511 can not only seal proximal blood flow, but also further meet requirements for exhaust of the inner sheath core 31. In other embodiments, the proximal end of the sealing tube 51b may also be fixedly connected to the proximal end of the inner sheath core 31 by gluing or welding or in other ways.

The design of the sealing tube 51b is to introduce the blood flow from a lumen of the outer sheath assembly 1 into a lumen formed between the inner sheath assembly 3 and the sealing tube 51b in a drainage way, which can not only avoid the blood from flowing out from a proximal end of the outer sheath assembly 1, but also achieve the purpose of simplifying the sealing structure. In addition, the horn mouth design of the guide piece 511b can not only achieve sealing by line contact, but also weaken movement resistance, strengthened by sealing, of the outer sheath assembly 1 to the maximum while achieving the good sealing effect.

As shown in FIG. 7, a delivery system 100 provided by an embodiment III of the present embodiment includes: an outer sheath assembly 1, an inner sheath assembly 3, a first sealing piece 51, a push rod assembly 7, and a guide rod 9. The embodiment III is different from the embodiment I mainly in a specific structure of the first sealing piece 51.

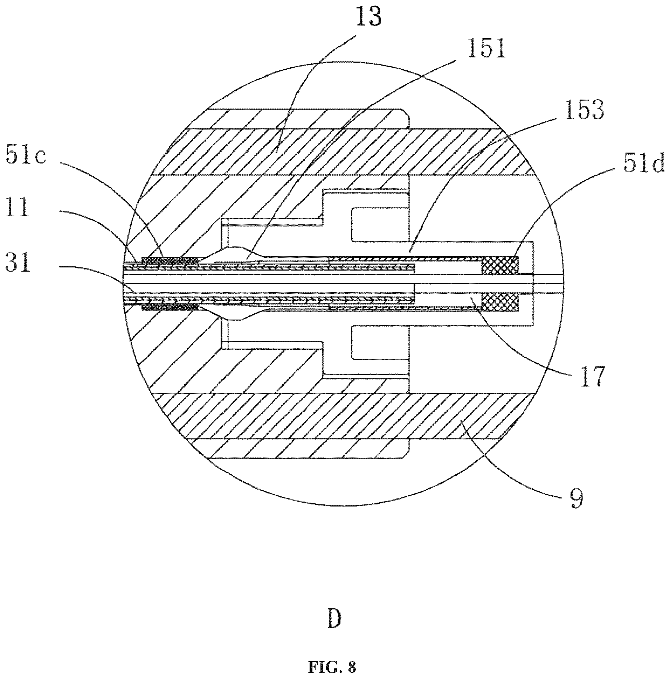

As shown in FIGS. 7 and 8, in the embodiment, an outer sheath core tube 11 is fixed to a proximal releaser 13 by a second connecting component 15. A cavity 17 facilitating adjustment on a proximal position of the outer sheath core tube 11 is formed between the proximal releaser 13 and the second connecting component 15. The cavity 17 is located between the proximal releaser 13 and an outer core lock 153. In the embodiment, a first sealing piece 51 is capable of sealing the cavity 17.

In the embodiment, two first sealing pieces 51 are arranged. The two first sealing pieces 51 are close to or located at a distal end of the proximal releaser 13 and a proximal end of the outer core lock 153 respectively. Specifically, one first sealing piece 51 is a proximal releaser sealing gasket 51c arranged at the distal end of the proximal releaser 13. The other first sealing piece 51 is an outer core lock sealing gasket 51d arranged at the proximal end of the outer core lock 153. It is to be understood that, in other embodiments, three, four or more first sealing pieces 51 may also be arranged, and the number may be set as actually needed.

By additionally arranging the proximal releaser sealing gasket 51c and the outer core lock sealing gasket 51d, a completely closed inner cavity is formed between the proximal releaser 13 and the outer core lock 153. The outer core lock sealing gasket 51d is positioned by the outer core grasper 151 and an end surface of an inner cavity of the outer core lock 153, so as to avoid the outer core lock sealing gasket 51d from moving in an axial direction. As a braided mesh tube or a spring tube is arranged in a tube wall of a sheath tube receiving the outer sheath core tube 11, the sheath tube needs to be machined by the steps of cleaning, coating with a hydrophilic coating, etc. before being assembled with the outer sheath core tube 11, the inner sheath core 31 and other elements together. In these machining processes, the sheath tube may be shortened or elongated, so that the shortened or elongated sheath tube needs to use the outer sheath core tube 11 of different lengths. Also because the length of the outer sheath core tube 11 used by the sheath tube of the same length specification is generally a fixed value, it is also unlikely to truncate or stretch the outer sheath core tube 11, thereby lowering a matching degree between the outer sheath core tube 11 and the sheath tube. In the present embodiment, by forming the cavity 17 facilitating adjustment on the proximal position of the outer sheath core tube 11 between the proximal releaser 13 and the second connecting component 15, a sufficient space for an axial change of the outer sheath core tube 11 is reserved in the inner cavity enclosed by the proximal releaser 13 and the outer core lock 153, thereby achieving the purpose of adapting to these micro-deformed sheath tubes, improving the assembly accuracy of the delivery system, and simplifying assembly steps. After flowing out from the gap between the outer sheath core tube 11 and the inner sheath core 31 through a proximal end of the outer sheath core tube 11, the blood flow enters the cavity 17. After the cavity 17 is fully filled, the blood flow may only enter a gap between the outer core grasper 151 and the outer sheath core tube 11. Just because of the presence of the proximal releaser sealing gasket 51c and the outer core lock sealing gasket 51d, the blood can only flow in a sealed cavity formed between the two sealing gaskets 51d and 51c, and no longer flows outside from the proximal end of the outer sheath core tube 11.

The proximal releaser sealing gasket 51c is located near the distal end of the outer core grasper 151, and is fitted to the outer sheath core tube 11. The proximal releaser sealing gasket 51c sleeves the outer sheath core tube 11. The inside diameter of the proximal releaser sealing gasket 51c should not be too large or too small. If it is too large, it is prone to strengthening resistance on axial movement of the outer sheath core tube 11; and if it is too small, it may lead to a poor sealing effect.

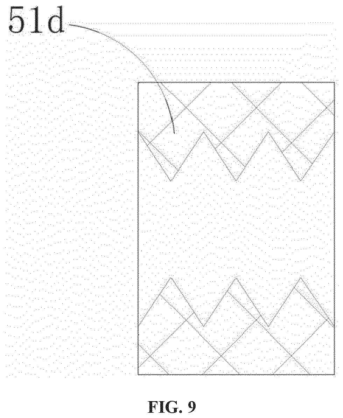

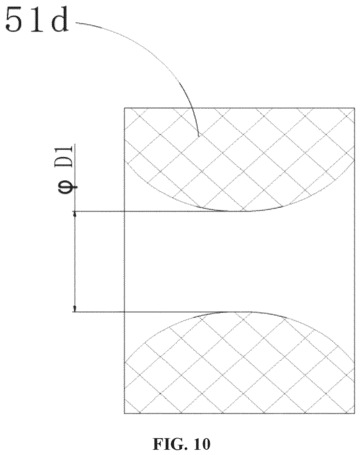

Relative movement is required between the outer core lock sealing gasket 51d and the inner sheath core 31. A selected structure of the outer core lock sealing gasket 51d may be a serrated surface as shown in FIG. 9, or an arc surface as shown in FIG. 10.

Taking the arc surface in FIG. 10 as an example, an inside diameter D1 of the outer core lock sealing gasket 51d and an outside diameter d1 of the inner sheath core 31 in FIG. 7 satisfy the following relationship:

$$\text{\O}D1=(1.1\text{-}1.5)\,\text{\O}d1$$

It is because the outer core lock sealing gasket 51d makes line contact with the inner sheath core 31. By using this contact way, the resistance caused by the outer core lock sealing gasket 51d on the inner sheath core 31 may be weakened while the sealing effect is achieved.

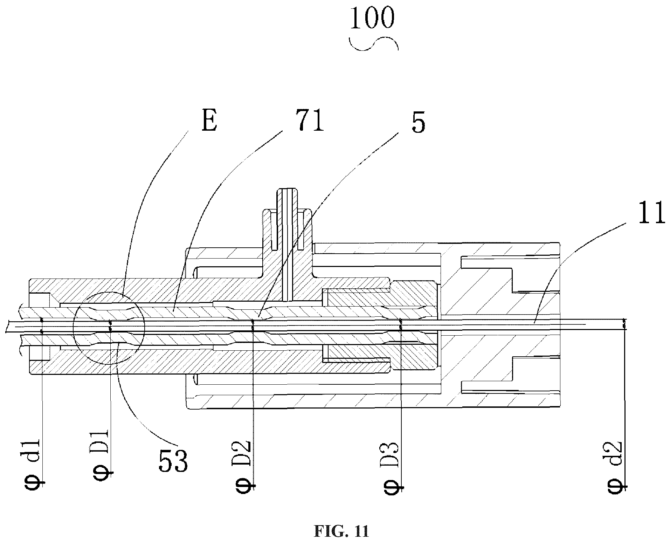

As shown in FIG. 11, a delivery system 100 provided by an embodiment IV of the present embodiment is different from the embodiment I in that: a sealing assembly 5 further includes a second sealing piece 53 in addition to a first sealing piece (not shown in the FIG. 11). In the embodiment, the first sealing piece may be of a structure of any one of the first sealing pieces 51 in embodiments 1-3, which will not be elaborated here.

As shown in FIG. 11, in the embodiment, the second sealing piece 53 performs sealing for blood leakage between an outer sheath core tube 11 and an inner cavity of a push rod 71. The second sealing piece 53 is capable of sealing a gap between the outer sheath core tube 11 and the push rod 71.

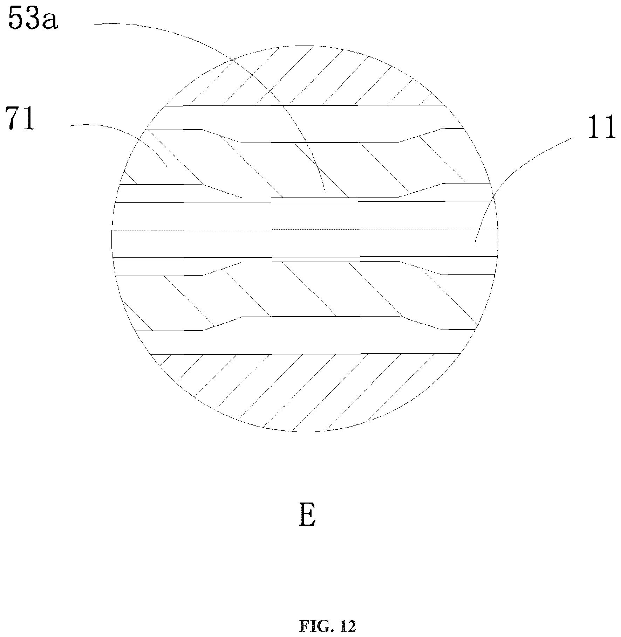

The second sealing piece 53 may be of a plurality of structures. As shown in FIG. 12, in the embodiment, the second sealing piece 53 includes at least one bulge 53a. The bulge 53a is located between the outer sheath core tube 11 and the push rod 71, so as to decrease the gap between the outer sheath core tube 11 and the push rod 71. In the embodiment, the bulge 53a is arranged on the push rod 71, and forms an integral structure with the push rod 71. Specifically, in the embodiment, the push rod 71 is made of a polymer thermoplastic material. The push rod 71 is heated to shrink to form a shrink mouth which is the bulge 53a. In other embodiments, the bulge 53a and the push rod 71 may also be separately formed, and then fixedly connected together by gluing or screwing or in other ways.

As shown in FIG. 11, in order to improve a sealing effect, a plurality of bulges 53a are arranged in the embodiment. The plurality of bulges 53a are arranged in an axial direction of the push rod 71.

Inside diameter dimensions of the bulges 53a change regularly. From a distal end of the push rod 71 to a proximal end of the push rod 71, radial distances between the bulges 53a and the outer sheath core tube 11 are gradually decreased. That is, from a distal end of the outer sheath core tube 11 to a proximal end of the outer sheath core tube 11, inner walls of the bulges 53a gradually approach an outer wall of the outer sheath core tube 11.

As shown in FIG. 11, an inside diameter of the push rod is d1, and an outside diameter of the outer sheath core tube 11 is d2. In the embodiment, three bulges 53a are sequentially arranged from the distal end of the push rod 71 to the proximal end of the push rod 71, having inside diameters of D1, D2 and D3 respectively, where the inside diameter d1 of the push rod, the outside diameter d2 of the outer sheath core tube 11, and the inside diameters D1, D2 and D3 of the three bulges 53a satisfy the following relationship:

$$\text{\O}d1>\text{\O}D1>\text{\O}D2>\text{\O}D3>\text{\O}d2$$

The inside diameters of the bulges 53a are gradually decreased from the distal end of the push rod 71 to the proximal end of the push rod 71. There is a certain interval between every two bulges 53a. The purpose of this design is that if a blood flow passes through the first bulge 53a, a blood flow volume is decreased by resistance due to decrease in the gap. After the blood flow passes through a normal gap section, pressure of the blood flow is decreased at this time; and then after the blood passes through a next bulge 53a with a smaller gap, the blood flow volume and the pressure of the blood flow are further decreased. Through obstruction of the plurality of bulges 53a with different inside diameters, the blood flow volume and the pressure of the blood flow are gradually decreased, so as to achieve the sealing effect. The method is simple to operate, and easy to realize by arranging the bulges 53a in local positions, and the dimensional accuracy is easy to control. Structural design of the bulges 53a only reduces the local gap between the outer sheath core tube 11 and the inner cavity of the push rod 71, which less affects circumferential movement of the outer sheath core tube 11. It will not significantly strengthen resistance on circumferential movement of the outer sheath core tube 11, and there is no newly added part, so that the good sealing effect can be achieved, and the risk of blood leakage is reduced.

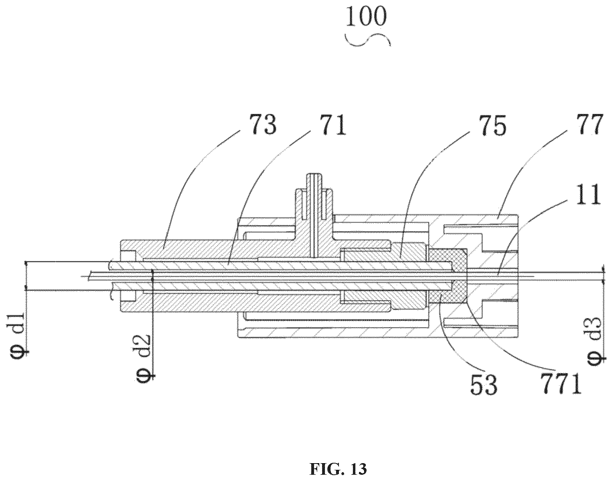

As shown in FIG. 13, it is a delivery system 100 provided by embodiment V of the present embodiment, which is different from that in the embodiment IV mainly in a specific structure of a second sealing piece 53. In the embodiment, a first sealing piece (not shown in the FIG. 13) may be of a structure of any one of the first sealing pieces 51 in embodiments 1-3, which will not be elaborated here.

In the embodiment, the second sealing piece 53 is located between an outer sheath core tube 11 and a push rod 71, to be used for blocking a gap between the outer sheath core tube 11 and the inner cavity of the push rod 71. A clamping groove 771 with an opening facing a distal end is formed in a rear handle 77, corresponding to the push rod 71. The second sealing piece 53 is arranged in the clamping groove 771.

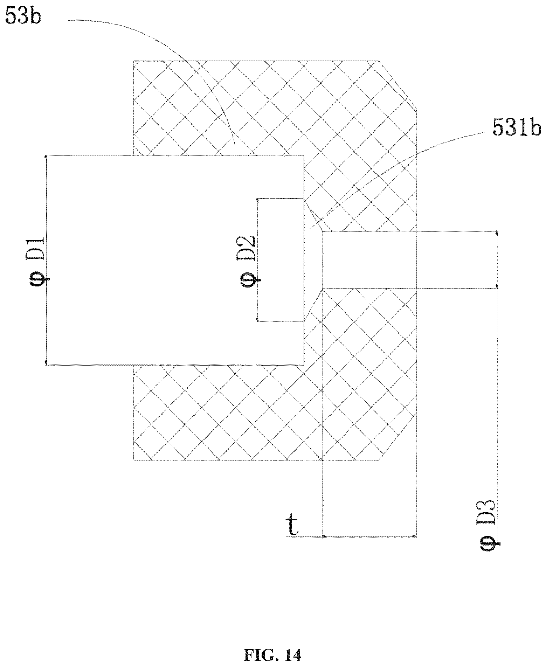

As shown in FIGS. 13 and 14, specifically, the second sealing piece 53 includes a push rod sealing gasket 53b. The push rod sealing gasket 53b is arranged at a proximal end of the push rod 71. In the embodiment, the push rod 71 extends out of a proximal edge of a third connecting component 75. The push rod sealing gasket 53b sleeves the extended push rod 71. The push rod 71 and the push rod sealing gasket 53b are inserted into the clamping groove 771 of the rear handle 77 together. The push rod sealing gasket 53b is fixed to the rear handle 77 by the push rod 71.

As shown in FIG. 14, a void avoidance groove 531b is formed in a position, close to the third connecting component 75, of the push rod sealing gasket 53b. An inside diameter of the void avoidance groove 531b is gradually decreased from a distal end of the outer sheath core tube 11 to a proximal end of the outer sheath core tube 11.

As shown in FIGS. 13 and 14, the push rod sealing gasket 53b needs to satisfy a fitting relationship with the push rod 71, the outer sheath core tube 11 and an inner sheath core 31: an inside diameter D1 of the push rod sealing gasket 53b and an outside diameter d1 of the push rod 71 satisfy the following relationship:

$$\varnothing D1 < \varnothing d1.$$

It is necessary to keep a certain magnitude of interference for fitting the push rod sealing gasket 53b to a hole shaft of the push rod 71, so as to achieve a sealing effect; a maximum inside diameter D2 of the void avoidance groove 531b and the inside diameter d2 of the push rod 71 satisfy the following relationship:

$$\varnothing D2 > \varnothing d2$$

Here is a void avoidance structure. The maximum inside diameter D2 of the void avoidance groove 531b is larger than the inside diameter d2 of the push rod 71, so as to prevent the situation that the soft push rod sealing gasket 53b is stuck into a gap between the outer sheath core tube 11 and the push rod 71 when the outer sheath core tube 11 axially moves relative to the push rod 71, and then a friction between the above two is strengthened.

An inside diameter D3 of the push rod sealing gasket 53b and an outside diameter d3 of the outer sheath core tube 11 satisfy the following relationship:

$$\varnothing d3 = (1.1\text{-}1.5)\, \varnothing D3$$

That is, the outside diameter of the outer sheath core tube 11 is larger than or equal to 1.1 times of the inside diameter of the push rod sealing gasket 53b, and is smaller than or equal to 1.5 times of an inner diameter of the push rod sealing gasket 53b. This is because fit between the outside diameter of the outer sheath core tube 11 and a through hole of the push rod sealing gasket 53b cannot be too large or too small. If it is too large, it is easy to strengthen resistance on axial movement of the outer sheath core tube 11; and if it is too small, it may lead to the poor sealing effect.

A length t of a sealing section of the push rod sealing gasket 53b satisfies the following relationship:

$$t = (1\text{-}3)\, mm$$

That is, the length of the sealing section is larger than or equal to 1 mm, and smaller than or equal to 3 mm. This is because the length of the sealing section of the push rod sealing gasket 53b should not be too small or too large. If it is too small, it may lead to the poor sealing effect; and if it is too large, it may strengthen the resistance on axial movement of the outer sheath core tube 11.

In the embodiment, by structural design with the additional arrangement of the push rod sealing gasket 53b, the gap between the outer sheath core tube 11 and the inner cavity of the push rod 71 may be completely blocked, so as to achieve a complete sealing effect.

During use of the delivery system 100, the outer sheath assembly 1 slides in the axial direction of the guide rod 9 under the external force, so as to release a stent. The inner sheath core 31 remains stationary during axis movement of the outer sheath core tube 11. In order to ensure smoothness of movement of the outer sheath assembly 1, there is a certain gap between the outer sheath core tube 11 and the inner sheath core 31. During a surgery, the blood flowing out along a gap between the outer sheath core tube 11 and the inner sheath core 31 is hindered by the first sealing piece 51, which effectively avoids the blood from flowing out from the proximal end of the outer sheath core tube 11. In addition, the blood flowing out along the gap between the outer sheath core tube 11 and the inner cavity of the push rod 71 is hindered by the second sealing piece 53, which further avoids the blood from flowing out from the proximal end of the outer sheath core tube 11.

Sealing performance of the existing delivery system mainly depends on decrease in a gap between the components for sealing. As length tolerance of each component of the delivery system is difficult to control accurately, it is difficult to meet the requirements for mass production by using a conventional sealing structure. The delivery system 100 of the present embodiment solves the problem of blood leakage of a product, can also ensure normal movement of a moving component at the same time, and can allow the length of each component to have a large dimensional tolerance, which reduces tolerance requirements for manufacturing to a certain extent and lowers the production cost.

It is to be understood that a sealing principle between an inner sheath core tube and the outer sheath core tube can also be adopted for sealing between the outer sheath core tube and the push rod as actually needed, so as to achieve sealing between the outer sheath core tube and the push rod; and a sealing principle between the outer sheath core tube and the push rod can also be adopted for sealing between the inner sheath core tube and the outer sheath core tube as actually needed, so as to achieve sealing between the inner and outer sheath core tubes.

The embodiments described above represent only a few embodiments of the present disclosure, the description of which is specific and detailed, but should not be construed to limit the scope of the present disclosure. It can be noted that several variations and modifications may be made by those skilled in the art without departing from the spirit of the present disclosure, which all fall within the scope of the present disclosure. Therefore, the protection scope of the patent for the present disclosure shall be determined by the appended claims.

## Claims

1. A delivery system, comprising an outer sheath assembly, an inner sheath assembly, and a sealing assembly, wherein the outer sheath assembly comprises an outer sheath core tube; the inner sheath assembly comprises an inner sheath core arranged in the outer sheath core tube in a penetrating manner; the sealing assembly comprises a first sealing piece; and the first sealing piece is arranged between the inner sheath core and the outer sheath core tube to seal a gap between a proximal end of the outer sheath core tube and the inner sheath core.

2. The delivery system according to claim 1, wherein the first sealing piece extends in a length direction of the outer sheath core tube.

3. The delivery system according to claim 2, wherein the first sealing piece comprises at least one sealing ring, the sealing ring is connected to the inner sheath core or the outer sheath core tube, and the sealing ring is arranged between the outer sheath core tube and the inner sheath core.

4. The delivery system according to claim 3, wherein a plurality of sealing rings are arranged in an axial direction of the inner sheath core; and the sealing rings sleeve outside the inner sheath core, and from a distal end of the inner sheath core to a proximal end of the inner sheath core, radial distances between the sealing rings and the outer sheath core tube are gradually decreased.

5. The delivery system according to claim 2, wherein each first sealing piece comprises a sealing tube, and a proximal end of the sealing tube is connected to that of the inner sheath assembly.

6. The delivery system according to claim 5, wherein the sealing tube is arranged in the outer sheath core tube in a penetrating manner; the inner sheath assembly further comprises a rear connecting piece and a first connecting component; the inner sheath core is fixed to the rear connecting piece by the first connecting component, and a proximal sealing piece is arranged at a proximal end of the first connecting component; and the proximal end of the sealing tube penetrates through the rear connecting piece to be in sealing connection with the proximal sealing piece of the first connecting component.

7. The delivery system according to claim 6, wherein a guide piece is arranged at a distal end of the sealing tube, from the distal end of the sealing tube to the proximal end of the sealing tube, an inside diameter of the guide piece is gradually decreased, the guide piece is used for introducing blood flow into a space between the inner sheath core and the sealing tube, and a distal end of the guide piece is in interference fitting to the outer sheath core tube.

8. The delivery system according to claim 1, wherein the outer sheath assembly further comprises a proximal releaser and a second connecting component; the outer sheath core tube is fixed to the proximal releaser by the second connecting component; and a cavity facilitating adjustment on a distal position of the outer sheath core tube is formed between the proximal releaser and the second connecting component, and the first sealing piece is capable of sealing the cavity.

9. The delivery system according to claim 8, wherein the second connecting component comprises an outer core grasper and an outer core lock, and the outer core lock drives the outer core grasper to fix the outer sheath core tube to the proximal releaser; and the cavity is located between the proximal releaser and the outer core lock, two first sealing pieces are arranged, and the two first sealing pieces are close to or located at a distal end of the proximal releaser and a proximal end of the outer core lock respectively.

10. The delivery system according to claim 1, wherein the delivery system further comprises a push rod assembly, the push rod assembly comprises a push rod, and the outer sheath core tube is arranged in the push rod in a penetrating manner; and the sealing assembly further comprises a second sealing piece, and the second sealing piece is capable of sealing a gap between the outer sheath core tube and the push rod.

11. The delivery system according to claim 10, wherein the second sealing piece comprises at least one bulge, the bulge is arranged on the push rod, protrudes in a direction close to the outer sheath core tube, and is located between the outer sheath core tube and the push rod.

12. The delivery system according to claim 11, wherein a plurality of bulges are arranged in an axial direction of the push rod, and from a distal end of the push rod to a proximal end of the push rod, radial distances between the bulges and the outer sheath core tube are gradually decreased.

13. The delivery system according to claim 10, wherein the push rod assembly further comprises a push rod joint and a third connecting component, the push rod is fixed to the push rod joint by the third connecting component, and the second sealing piece is located between the outer sheath core tube and the push rod.

14. The delivery system according to claim 13, wherein the push rod assembly further comprises a rear handle, the rear handle is connected to the third connecting component, a proximal end of the push rod is inserted into the third connecting component, a clamping groove is formed in the rear handle, corresponding to the push rod, and the second sealing piece is arranged in the clamping groove.

15. The delivery system according to claim 14, wherein the second sealing piece comprises a push rod sealing gasket, a void avoidance groove is formed in the push rod sealing gasket close to the third connecting component, and from a distal end of the outer sheath core tube to the proximal end of the outer sheath core tube, an inside diameter of the void avoidance groove is gradually decreased.

FIG. 1

A

**FIG. 2**

FIG. 3

FIG. 4

C

FIG. 5

**FIG. 6**

**FIG. 7**

D

**FIG. 8**

**FIG. 9**

51d

φ D1

FIG. 10

100

E    71    5

11

φ d1    φ D1    53    φ D2    φ D3    φ d2

FIG. 11

53a

71

11

E

FIG. 12

FIG. 13

**FIG. 14**

## INTERNATIONAL SEARCH REPORT

| | International application No. |
|---|---|
| | **PCT/CN2022/136076** |

**A.    CLASSIFICATION OF SUBJECT MATTER**

A61F2/07(2013.01)i

According to International Patent Classification (IPC) or to both national classification and IPC

**B.    FIELDS SEARCHED**

Minimum documentation searched (classification system followed by classification symbols)

A61F2/-

Documentation searched other than minimum documentation to the extent that such documents are included in the fields searched

Electronic data base consulted during the international search (name of data base and, where practicable, search terms used)

WPI, EPODOC, CNPAT, CNKI: 先健, 肖本好, 唐江峰, 输送, 递送, 鞘, 管, 近侧, 近端, 流, 漏, 血, 密封, 内, 外, 芯, 心, deliver +, convey+, sheath, catheter, proximal, leakage, blood, seal+, inner, outer, core

**C.    DOCUMENTS CONSIDERED TO BE RELEVANT**

| Category* | Citation of document, with indication, where appropriate, of the relevant passages | Relevant to claim No. |
|---|---|---|
| PX | CN 217066706 U (LIFETECH SCIENTIFIC (SHENZHEN) CO., LTD.) 29 July 2022 (2022-07-29) description, paragraphs [0043]-[0111], and figures 1-14 | 1-15 |
| X | CN 211674738 U (SHANGHAI LANMAI MEDICAL TECHNOLOGY CO., LTD.) 16 October 2020 (2020-10-16) description, paragraphs [0076]-[0097], and figures 1-10 | 1-15 |
| X | US 2012041532 A1 (COOK MEDICAL TECHNOLOGIES L.L.C.) 16 February 2012 (2012-02-16) description, paragraph [0034], and figure 4 | 1-4 |
| A | CN 206499552 U (SHANGHAI MICROPORT CARDIOFLOW MEDTECH CO., LTD.) 19 September 2017 (2017-09-19) entire document | 1-15 |
| A | CN 110769887 A (EDWARDS LIFESCIENCES CORP.) 07 February 2020 (2020-02-07) entire document | 1-15 |

☑ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 February 2023** | **20 February 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
|---|
| **PCT/CN2022/136076** |

**C.   DOCUMENTS CONSIDERED TO BE RELEVANT**

| Category* | Citation of document, with indication, where appropriate, of the relevant passages | Relevant to claim No. |
|---|---|---|
| A | CN 108245290 A (LIFETECH SCIENTIFIC (SHENZHEN) CO., LTD.) 06 July 2018 (2018-07-06)<br> entire document | 1-15 |
| A | CN 113521492 A (LIFETECH SCIENTIFIC (SHENZHEN) CO., LTD.) 22 October 2021 (2021-10-22)<br> entire document | 1-15 |
| A | US 2008116647 A1 (INSULET CORP.) 22 May 2008 (2008-05-22)<br> entire document | 1-15 |

Form PCT/ISA/210 (second sheet) (July 2022)

INTERNATIONAL SEARCH REPORT

Information on patent family members

International application No.

**PCT/CN2022/136076**

| Patent document cited in search report | | | Publication date (day/month/year) | Patent family member(s) | | | Publication date (day/month/year) |
|---|---|---|---|---|---|---|---|
| CN | 217066706 | U | 29 July 2022 | None | | | |
| CN | 211674738 | U | 16 October 2020 | CN | 110882096 | A | 17 March 2020 |
| | | | | WO | 2021128940 | A1 | 01 July 2021 |
| | | | | KR | 20220119679 | A | 30 August 2022 |
| US | 2012041532 | A1 | 16 February 2012 | US | 8585748 | B2 | 19 November 2013 |
| CN | 206499552 | U | 19 September 2017 | None | | | |
| CN | 110769887 | A | 07 February 2020 | WO | 2018236953 | A1 | 27 December 2018 |
| | | | | US | 2018368979 | A1 | 27 December 2018 |
| | | | | US | 10639152 | B2 | 05 May 2020 |
| | | | | US | 2020261227 | A1 | 20 August 2020 |
| | | | | JP | 2020524571 | A | 20 August 2020 |
| | | | | JP | 7187495 | B2 | 12 December 2022 |
| | | | | EP | 3641868 | A1 | 29 April 2020 |
| | | | | KR | 20200010580 | A | 30 January 2020 |
| CN | 108245290 | A | 06 July 2018 | CN | 207071112 | U | 06 March 2018 |
| CN | 113521492 | A | 22 October 2021 | None | | | |
| US | 2008116647 | A1 | 22 May 2008 | US | 7771412 | B2 | 10 August 2010 |

Form PCT/ISA/210 (patent family annex) (July 2022)